# FASCICULE DE BREVET EUROPEEN

(11) **EP 2 485 680 B1**
(45) Date de publication et mention de la délivrance du brevet: **04.03.2015**
(21) Numéro de dépôt: 10775703.1
(22) Date de dépôt: 06.10.2010
(51) Int. Cl.: A61F 2/01

(54) **CARTOUCHE DE SECURITE POUR UN FILTRE CAVE RETIRABLE**
SICHERHEITSKARTUSCHE FÜR EINEN ENTNEHMBAREN HOHLVENENFILTER
SAFETY CARTRIDGE FOR A REMOVABLE VENA CAVA FILTER

(30) Priorité: 06.10.2009 FR 0904780
(43) Date de publication de la demande: 15.08.2012
(73) Titulaire: B. Braun Medical Sas, 92660 Boulogne Billancourt Cedex (FR)
(72) Inventeur: VIGNAUD, Alain, F-86190 Quinçay (FR); GUTKNECHT, Dan, F-86130 Jaunay Clan (FR); FORBER, Simon, F-86240 Liguge (FR)
(74) Mandataire: Winter, Brandl, Fürniss, Hübner, Röss, Kaiser, Polte - Partnerschaft mbB
(86) Numéro de dépôt international: PCT/EP2010/006096
(87) Numéro de publication internationale: WO 2011/042163

(56) Documents cités:
- WO-A1-2007/021340
- WO-A2-2005/107639

## Description

La présente invention concerne une cartouche de sécurité pour un filtre cave retirable.

On utilise des filtres caves pour la prévention des embolies pulmonaires. A cette fin, on introduit dans la veine un filtre pliable au moyen d'un cathéter, le plus souvent dans la veine cave inférieure afin d'éviter que des caillots sanguins puissent remonter vers l'artère pulmonaire. Les filtres retirables sont conçus pour être utilisés temporairement, par exemple, lors d'interventions chirurgicales, et pour être ensuite retirés. Ils peuvent être également laissés à demeure dans le patient si son état nécessite une protection permanente

Les filtres sont pliables de sorte qu'ils puissent être introduits pliés dans la lumière interne d'un cathéter et placés dans une veine au moyen du dit cathéter. L'extrémité distale du cathéter est positionnée à l'endroit d'insertion souhaité pour le filtre et ce dernier est alors sorti du cathéter. A cet effet, le filtre est généralement maintenu dans sa position par rapport à la veine grâce à un dispositif de maintien en appui sur son extrémité proximale et le cathéter est retiré progressivement. Le filtre plié qui se trouve dans le cathéter se déplie dès qu'il sort de l'extrémité distale du cathéter et se déploie dans la veine.

Un type de filtre courant est composé de jambes sous forme de fils métalliques susceptible d'être ouverts sous forme d'éventail, regroupés sur l'extrémité proximale. A l'état plié, à l'intérieur du cathéter, les jambes sont sous tension et orientés à peu près parallèlement les unes par rapport aux autres. A l'extérieur du cathéter, du fait de leur élasticité, les jambes s'écartent pour que le filtre puisse se déployer dans la veine. Quelques jambes au moins ont, sur leurs extrémités, des crochets barbelés avec lesquels le filtre peut s'ancrer dans les parois du vaisseau sanguin.

Pour être retiré, le filtre dispose d'un crochet sur son extrémité proximale ou d'un autre moyen de préhension. On peut alors introduire un cathéter dans la veine jusqu'à la position du filtre. Le filtre est capturé par un dispositif correspondant, qui peut être un « lasso » ou un autre système adapté. Pour que le filtre se détache des parois des vaisseaux, on continue à pousser le cathéter dans la direction distale, le filtre étant maintenu dans sa position par le dispositif de capture. Plus le filtre est recouvert par le cathéter, plus ses jambes sont comprimées. Les extrémités des jambes et les crochets barbelés se détachent de la paroi du vaisseau et le filtre peut être logé complètement dans le cathéter, et ensuite retiré par celui-ci dans la direction proximale.

Pour que les crochets barbelés ne s'ancrent pas à l'extrémité distale du cathéter, les jambes munis de crochets barbelés sont en général légèrement repliés vers l'intérieur sur l'extrémité, de sorte que les crochets barbelés ne fassent pas saillie sur le contour extérieur lorsque le filtre est plié et ne puissent pas s'ancrer sur le cathéter.

Selon l'état de la technique, on retire le filtre totalement de la lumière interne du cathéter pour que ce dernier, resté dans la veine, puisse être utilisé avant son retrait pour d'autres examens. Dans ces conditions, le filtre se déploie dès sa sortie du cathéter et expose à l'air libre ses moyens de fixation. Du fait de leur agressivité, les crochets barbelés peuvent blesser par piqure le personnel soignant. De la même façon, le filtre peut, en se déployant brutalement, provoquer des projections de sang qui peut être contaminé. Il existe de ce fait un risque élevé de contamination au contact de matériel souillé par le sang, soit par piqure, soit par projection.

Le document WO 2007/021340 A1 divulgue le préambule de la revendication 1.

Le but de la présente invention est de réaliser un dispositif de retrait d'un filtre cave retirable d'un vaisseau sanguin, qui diminue le risque de blessure pour le personnel soignant.

Cette tâche est résolue grâce à une cartouche dite de sécurité selon la revendication 1. On peut trouver des modes de réalisation avantageux dans les revendications dépendantes 2 à 7.

La cartouche de sécurité conforme à l'invention pour des filtres caves retirables, comporte une extrémité proximale, une extrémité distale et une lumière interne qui s'étend entre les extrémités. Elle est caractérisée en ce qu'elle présente sur son extrémité distale, un dispositif de liaison pour la relier à un cathéter. Elle présente, en outre, dans sa lumière interne, une saillie de dimension limitée dans la direction axiale de la cartouche pour diminuer localement la section transversale de la lumière. Cette saillie est de préférence agencée espacée des extrémités de la cartouche.

Une cartouche de sécurité de ce type peut être raccordée avec son dispositif de liaison à l'extrémité proximale d'un cathéter veineux pour retirer un filtre cave. Le filtre peut alors être capturé de manière connue avec un dispositif conventionnel comme un lasso et rentré dans l'extrémité distale du cathéter. Au travers du cathéter, le filtre est ensuite introduit dans la cartouche qui est agencée sur l'extrémité proximale du cathéter. On tire l'extrémité proximale du filtre avec le dispositif de capture à travers la lumière de la cartouche et l'on passe la saillie. Cependant, les crochets des jambes orientés vers l'extérieur s'ancrent sur l'épaulement de la saillie et limitent le déplacement du filtre à l'intérieur de la cartouche. Le filtre ne peut plus être retiré de la cartouche du côté proximal. Le danger de blessure pour le personnel soignant est exclu. Il n'existe pas de danger de blessure sur les crochets ou par contamination par projection de sang.

La cartouche contenant le filtre connecté au système de retrait peut alors être séparée du cathéter moyennant le dispositif de liaison. Le cathéter peut rester dans la veine, par exemple, pour effectuer une cavographie de contrôle.

Indépendamment, le filtre peut être sorti de la cartouche en le poussant vers l'extrémité distale de cette dernière en utilisant le système de retrait. Le filtre peut alors être recueilli dans une boite de prélèvement, puis désolidarisé du système de retrait, qui peut par exemple être un lasso.

La cartouche de sécurité peut présenter sur son extrémité proximale un système d'étanchéité. Ce dernier peut être constitué d'une ou plusieurs valves hémostatiques ou être un système du type presse-étoupe. Un tel système peut par exemple être composé de deux valves montées en série. Dans ce cas, une première valve fendue assure l'étanchéité par le contact des deux lèvres en l'absence d'introduction du dispositif de retrait. Une seconde valve, présentant un perçage de diamètre approprié, assure quant à elle l'étanchéité lorsque le système de retrait la traverse. Le système permet alors l'étanchéité de la cartouche en présence et en l'absence du système de retrait.

Le dispositif de liaison pour relier la cartouche avec un cathéter peut être, par exemple, une liaison par vissage ou une liaison à baïonnette. Il peut également, de manière avantageuse, inclure une liaison « cône/cône » de type Luer.

La saillie peut être réalisée de façon annulaire de manière à s'étendre localement le long de toute la circonférence de la lumière. En fonction du filtre utilisé, il peut toutefois être aussi suffisant si la saillie ne s'étend que sur une partie de la circonférence. La saillie peut être continue ou discontinue.

De préférence, la saillie présente sur son côté distal un épaulement formant un angle inférieur à 150° avec le grand axe de la cartouche. Cet angle est de préférence inférieur à 135° et notamment approximativement rectangulaire. L'épaulement peut également présenter un angle inférieur à 90° sur son côté distal et notamment former un angle aigu avec le grand axe de la cartouche. De cette manière, les crochets sur les jambes du filtre peuvent s'ancrer dans la saillie et ne glissent pas. Le filtre est maintenu sûrement dans la cartouche et ne peut pas être tiré hors de la cartouche dans la direction proximale.

La cartouche est avantageusement dimensionnée de manière à ce que la distance entre la saillie et le système d'étanchéité de la cartouche soit supérieure à la longueur totale à l'état plié de la partie du filtre située entre son extrémité proximale et ses moyens d'ancrage. Le filtre reste ainsi entièrement contenu dans la cartouche, et seul le système de retrait passe à travers le système d'étanchéité. L'étanchéité de.la cartouche n'est alors pas compromise.

La cartouche peut être avantageusement une pièce moulée, fabriquée selon un procédé de moulage par injection.

Un exemple de réalisation de l'invention est décrit plus en détail dans ce qui suit à l'aide des figures jointes, dont :
- **Figure 1**: montre une cartouche de sécurité conforme à l'invention en section transversale ;
- **Figure 2**: montre un filtre cave retirable d'un type qui peut être retiré à l'aide de la cartouche de sécurité conforme à l'invention, lorsqu'il est déployé, dans une représentation en perspective ;
- **Figure 3**: montre une cartouche de sécurité conforme à l'invention dans une vue latérale en liaison avec un cathéter;
- **Figures 4a à 4f**: montrent le retrait progressif du filtre de la figure 2 d'un vaisseau sanguin ;
- **Figure 5**: montre une cartouche de sécurité selon la figure 1 en section transversale qui comporte un filtre retiré d'un vaisseau sanguin ;
- **Figures 6a et 6b**: montrent schématiquement, en section transversale, l'enlèvement d'un filtre de la cartouche ;
- **Figure 7**: montre, en section transversale, un système d'étanchéité comportant des valves pour l'extrémité proximale d'une cartouche ;
- **Figure 8**: montre, en section transversale, un système d'étanchéité de type presse-étoupe pour l'extrémité proximale d'une cartouche.

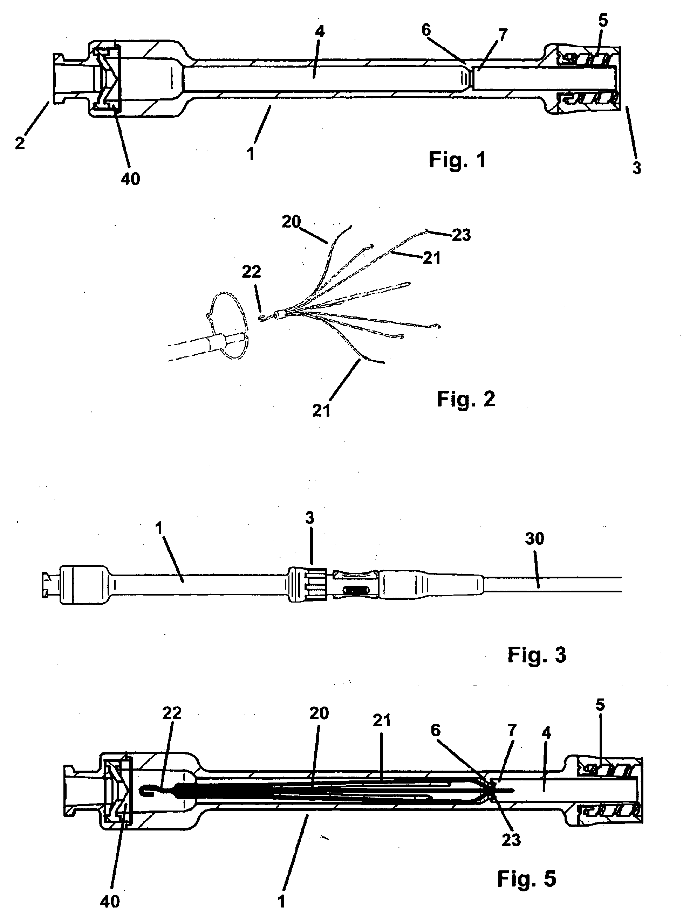

La **figure 1** montre une cartouche de sécurité **1** conforme à l'invention en section transversale. La cartouche **1** a une extrémité proximale **2** et une extrémité distale **3.** Une lumière interne **4,** dans cet exemple essentiellement cylindrique, s'étend entre les extrémités **2, 3.** La cartouche **1** présente sur son extrémité distale **3,** un filetage **5** pour relier la cartouche **1** avec un cathéter. Dans sa lumière **4,** la cartouche **1** présente une saillie **6** de dimension limitée en direction axiale de la cartouche qui diminue la lumière **4.** L'épaulement **7** de la saillie **6** sur le côté distal est perpendiculaire à la paroi intérieure de la lumière **4.** Sur le côté proximal, la saillie **6** est faiblement inclinée vers la paroi intérieure.

La cartouche de sécurité **1** présente, en outre, un système d'étanchéité **40** sur son extrémité proximale comportant deux valves hémostatiques.

La **figure 2** montre un filtre cave retirable **20** d'un type qui peut être retiré à l'aide de la cartouche de sécurité conforme à l'invention, déployé et dans une représentation en perspective. Le filtre **20** présente une multitude de jambes **21** qui sont regroupées dans un faisceau sur le côté proximal et s'écartent l'un par rapport à l'autre en direction distale lorsque le filtre se déploie. Le filtre **20** dispose sur le côté proximal d'un crochet **22.** Quelques-unes des jambes **21** possèdent sur leurs extrémités des crochets barbelés **23** qui sont orientés vers l'extérieur et s'ancrent dans la paroi du vaisseau pour positionner le filtre **20** de façon sûre.

La **figure 3** montre une cartouche de sécurité 1 conforme à l'invention dans une vue latérale en liaison avec un cathéter **30.** Le dispositif de liaison **3** permet la liaison amovible entre la cartouche **1** et le cathéter **30.**

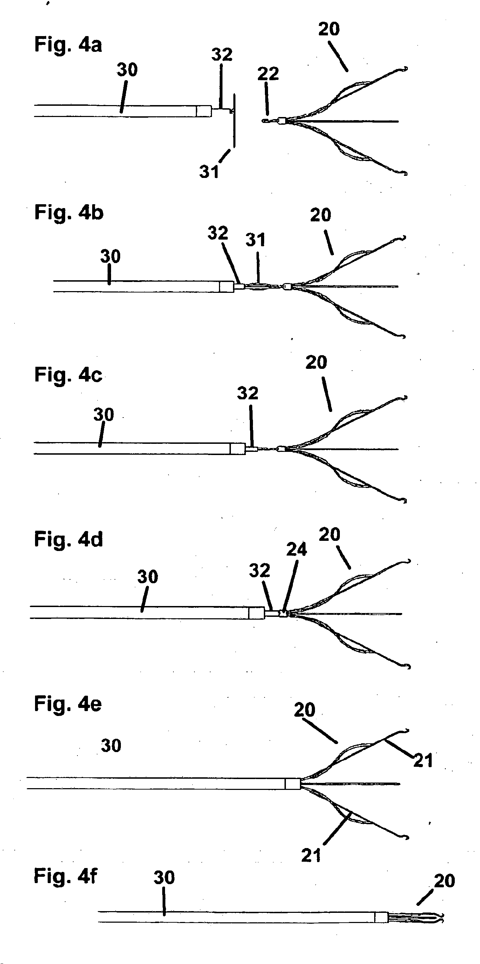

Les **figures 4a à 4f** montrent l'extrémité distale d'un cathéter **30** comportant un dispositif destiné à retirer un filtre cave retirable d'un vaisseau sanguin, en l'occurrence un lasso dans cette description, ainsi que le retrait progressif d'un filtre **20.** Le cathéter **30** comprend un fil métallique avec une boucle en fil métallique **31,** le dit lasso. Le fil métallique est guidé dans une gaine **32** se trouvant à l'intérieur du cathéter **30.** La gaine **32** peut se déplacer dans ledit cathéter. Le filtre à explanter doit être repéré. Ensuite, le cathéter **30** est introduit dans le vaisseau sanguin (non représenté) jusqu'à la position du filtre **20.** Le lasso **31** est libéré de l'extrémité distale du cathéter **30** (figure 4a). Avec le lasso **31** on capture le crochet **22** du filtre **20.** La gaine **32** est alors descendue en direction distale de manière à fermer le Lasso **31** (figure 4b). On continue à pousser la gaine **32** dans la direction distale (figure 4c) jusqu'à ce que le crochet soit réceptionné dans la gaine et que la tête **24** du filtre **20** soit maintenue de façon sûre (figure 4d). La gaine **32** est ensuite maintenue dans cette position et le cathéter **30** est bougé en direction distale de manière à se glisser au-dessus du filtre **20** (figure 4e) et que les jambes **21** du filtre se replient (figure 4f).

La **figure 5** montre une cartouche de sécurité 1 selon la figure 1 en section transversale qui comporte un filtre **20** explanté. On peut voir que le filtre **20** est tiré sur son crochet **22** dans la lumière **4** de la cartouche **1.** Les crochets barbelés **23** sur les jambes **21** du filtre s'ancrent ainsi dans l'épaulement **7** de la saillie **6.**

Les jambes **21** du filtre **20** portant les crochets barbelés **23** sont légèrement repliées vers l'intérieur sur leurs extrémités pour que les crochets **23** ne fassent pas saillie sur les contours du filtre lorsque le filtre **20** se replie et que le filtre puisse être introduit dans le cathéter. Il est donc avantageux que le contour de la saillie **6** suit le contour des jambes du filtre **21** pour assurer que les crochets **23** puissent s'ancrer correctement sur la saillie **6.**

La saillie **6** dans la lumière **4** est disposée de manière à ce que la distance entre la saillie **6** et le système d'étanchéité **40** de la cartouche soit supérieure à la longueur totale, à état plié, de la partie du filtre **20** située entre son extrémité proximale et ses moyens d'ancrage **23.** Le filtre **20** reste ainsi entièrement contenu dans la cartouche **1,** et seul le système de retrait passe à travers les valves du système d'étanchéité **40.** L'étanchéité de la cartouche **1** n'est alors pas compromise. La cartouche **1** peut être retirée du cathéter moyennant le dispositif de liaison **5** et éliminée. Il n'y pas de danger de se blesser pour le personnel soignant. Le cathéter **30** peut rester encore dans le vaisseau sanguin après le retrait du filtre **20** pour réaliser une cavographie de contrôle si besoin.

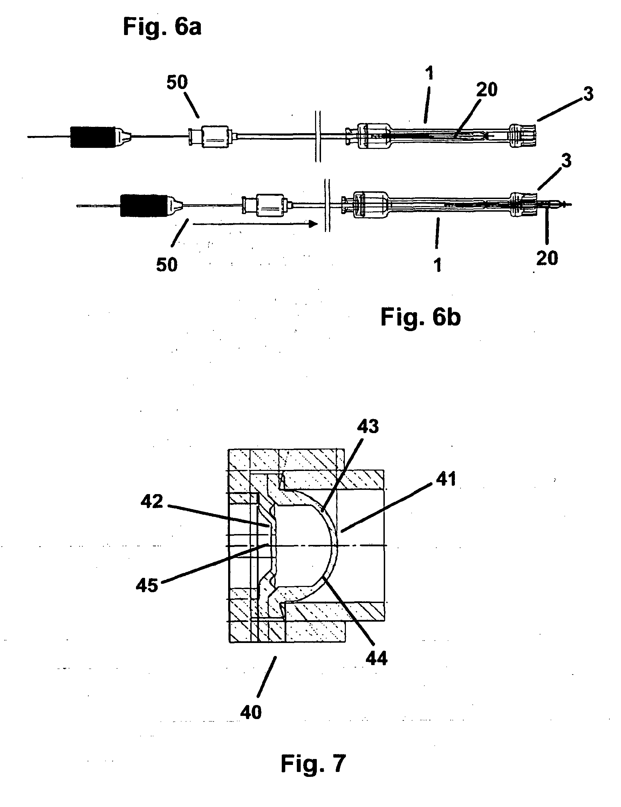

La **figure 6a** montre la cartouche **1** retirée du cathéter selon la figure 5 avec son système de retrait **40** pour le filtre **20.** Le filtre **20** peut être sorti de la cartouche 1 (figure 6b) en le poussant vers l'extrémité distale **3** de cette dernière en utilisant le système de retrait **40.** Le filtre peut alors être recueilli dans une boite de prélèvement (non représentée), puis désolidarisé du système de retrait **50,** qui peut par exemple être un lasso.

Le système d'étanchéité **40** représenté dans la **figure 7** est composé de deux valves **41, 42** montées en série. Une première valve **41** fendue assure l'étanchéité par le contact des deux lèvres **43, 44** en l'absence d'introduction du dispositif de retrait. Une seconde valve **42,** présentant un perçage **45** de diamètre approprié, assure quant à elle l'étanchéité lorsque le système de retrait la traverse. Le système **40** est monté sur l'extrémité proximale de la cartouche et permet alors l'étanchéité de la cartouche en présence et en l'absence du système de retrait.

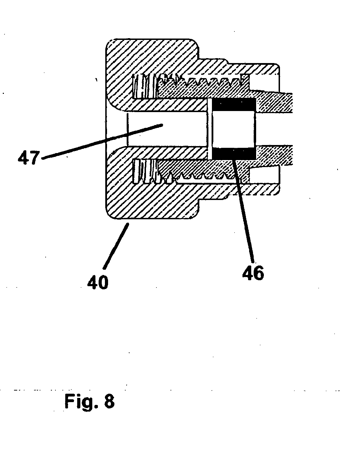

La **figure 8** montre, en section transversale, un système d'étanchéité **40** de type presse-étoupe. Le système comporte un joint torique **46** autour du passage **47** du système de retrait pour rendre le système étanche lorsque le système de retrait est en place.

## Revendications

1. Cartouche de sécurité (1) pour un filtre cave (20) retirable avec une extrémité proximale (2) et une extrémité distale (3) et avec une lumière interne (4) s'étendant entre les extrémités, présentant sur son extrémité distale (3) un dispositif de liaison (5) pour créer une liaison avec un cathéter (30), et
**caractérisée en ce que** la cartouche de sécurité (1) présente
- dans sa lumière interne une saillie (6) de dimension limitée dans la direction axiale de la cartouche (1) pour diminuer localement la section transversale de la lumière (4)
- la saillie étant agencée espacée des extrémités de la cartouche.

2. Cartouche de sécurité (1) selon la revendication 1, **caractérisée en ce que** la cartouche présente sur son extrémité proximale (2) un système d'étanchéité (40) comportant une ou plusieurs valves hémostatiques (41, 42) ou un système d'étanchéité de type presse-étoupe.

3. Cartouche de sécurité (1) selon la revendication 1 ou 2, **caractérisée en ce que** le dispositif de liaison (5) est une liaison par cône Luer, une liaison par vissage ou une liaison à baïonnette ou par la conjonction de plusieurs de ces éléments.

4. Cartouche de sécurité (1) selon l'une des revendications 1 à 3, **caractérisée en ce que** la saillie (6) présente sur le côté distal un épaulement (7) formant un angle inférieur à 150° avec le grand axe de la cartouche, de préférence un angle inférieur à 135°, notamment un angle approximativement rectangulaire ou un angle aigu.

5. Cartouche de sécurité (1) selon l'une des revendications 1 à 4, **caractérisée en ce que** la saillie (6) a une forme annulaire, continue ou discontinue.

6. Cartouche de sécurité (1) selon l'une des revendications 1 à 5, **caractérisée en ce que** la distance entre la saillie (6) et le système d'étanchéité (40) de la cartouche soit supérieure à la longueur totale de la partie d'un filtre conventionnel (20) située entre son extrémité proximale et ses moyens d'ancrage (23), le filtre (20) étant dans son état plié.

7. Cartouche de sécurité (1) selon l'une des revendications 1 à 6, **caractérisée en ce que** la cartouche est une pièce moulée.

## Patentansprüche

1. Sicherheitskartusche (1) für einen entnehmbaren Hülsenfilter (20) mit einem proximalen Ende (2) und einem distalen Ende (3) und mit einem inneren Licht (4), das sich zwischen den Enden erstreckt, enthaltend an seinem distalen Ende (3) eine Verbindungsvorrichtung (5) zum Herstellen einer Verbindung mit dem Katheter (30), und **dadurch gekennzeichnet, dass** die Sicherheitskartusche (1) bei dem internen Licht einen Vorsprung (6) einer begrenzten Ausdehnung in axialer Richtung der Kartusche (1) zum lokalen Verringern des Querschnitts des Lichts (4) aufweist, wobei ferner der Vorsprung von den Enden der Kartusche entfernt angeordnet ist.

2. Sicherheitskartusche (1) nach Anspruch 1, **dadurch gekennzeichnet, dass** die Kartusche an ihrem proximalen Ende (2) ein Abdichtungssystem (40) umfassend ein oder mehrere haemostatische Ventile (41, 42) oder ein Abdichtungssystem nach Art einer Verschraubung oder Stopfbuchse aufweist.

3. Sicherheitskartusche (1) nach Anspruch 1 oder 2, **dadurch gekennzeichnet, dass** der Verbindungsvorsprung (5) eine Verbindung über einen Luer-Konus, eine Verbindung über eine Verschraubung oder eine Verbindung über eine Bajonett-Verbindung oder eine Verbindung mehrerer dieser Elemente ist.

4. Sicherheitskartusche (1) nach einem der Ansprüche 1 bis 3, **dadurch gekennzeichnet, dass** der Vorsprung (6) an seiner distalen Seite eine Schulter (7) ausbildet, welche einen Winkel < 150° mit der Längsachse der Kartusche aufweist, vorzugsweise einen Winkel < 135°, insbesondere einen Winkel von ungefähr 90° oder einen spitzen Winkel.

5. Sicherheitskartusche (1) nach einem der Ansprüche 1 bis 4, **dadurch gekennzeichnet, dass** der Vorsprung (6) eine ringförmige, kontinuierliche oder diskontinuierliche Form aufweist.

6. Sicherheitskartusche (1) nach einem der Ansprüche 1 bis 5, **dadurch gekennzeichnet, dass** die Distanz zwischen dem Vorsprung (6) und dem Dichtsystem (40) der Kartusche größer ist, als die Gesamtlänge eines Abschnitts eines konventionellen Filters (20), welcher zwischen seinem proximalen Ende und Verankerungsmitteln (23) angeordnet ist, wobei der Filter (20) in seinem gebogenem Zustand ist.

7. Sicherheitskartusche (1) nach einem der Ansprüche 1 bis 6, **dadurch gekennzeichnet, dass** die Kartusche ein gegossenes Bauteil ist.

## Claims

1. Security cartridge (1) for a removable vena cava filter (20) with a proximal end (2) and a distal end (3) and with an internal light (4) which extends between the ends, including at its distal end (3) a connection device (5) for creating a connection with a catheter (30), and **characterized in that** the security cartridge (1) includes at its internal light a projection (6) of limited dimension in the axial direction of the cartridge (1) for diminishing locally the cross-section of the light (4) whereas the projection is spaced apart from the ends of the cartridge.

2. Security cartridge (1) according to claim 1, **characterized in that** the cartridge presents at its proximal end (2) a sealing system (40) comprising one or plural haemostatic valves (41, 42) or a sealing system of the screw plug- or packed valve type.

3. Security cartridge (1) according to claim 1 or 2, **characterized in that** the connection device (5) is a connection via a Luer cone, a connection via a screwing or a bayonet connection or a connection of multiple of these elements.

4. Security cartridge (1) according to one of the claims 1 to 3, **characterized in that** the projection (6) comprises at its distal side a shoulder (7) forming an angle smaller than 150° with the longitudinal axis of the cartridge, preferably an angle smaller than 135°, especially an angle approximately rectangular or an acute angle.

5. Security cartridge (1) according to one of the claims 1 to 4, **characterized in that** the projection (6) has an annular, continuous or discontinuous form.

6. Security cartridge (1) according to one of the claims 1 to 5, **characterized in that** the projection (6) and the sealing system (40) of the cartridge is superior to the total length of the part of a conventional filter (20) situated between the proximal end and its anchoring means (23), whereas the filter (20) is in a bent state.

7. Security cartridge (1) according to one of the claims 1 to 6, **characterized in that** the cartridge being a molded piece.
